# EUROPEAN PATENT APPLICATION

(11) **EP 4 748 913 A1**
(43) Date of publication of application: **27.05.2026**
(21) Application number: 23946538.8
(22) Date of filing: 21.07.2023
(51) Int. Cl.: C11C 3/00, C12P 7/6431, C12P 7/6436, A61K 31/232, A61P 17/00, A61K 33/00, A61K 8/22

(54) **HYPER-OZONISED FATTY ACIDS AND PRODUCTION METHOD THEREOF**

(71) Applicant: Biotelier Labs Healthcare S.L., 08474 Gualba Barcelona (ES)
(72) Inventor: ACEITUNO ALBERTI, Rodrigo Antonio, 08174 SANT CUGAT Barcelona (ES)
(74) Representative: Hoffmann Eitle
(86) International application number: PCT/ES2023/070479
(87) International publication number: WO 2025/022022

(57) **Abstract**

The present invention relates to a method for producing hyperozonized fatty acids from the high-grade ozonization of extra virgin oil comprising a high concentration of polyunsaturated fatty acids. Likewise, the present invention relates to the hyperozonized fatty acids produced by means of said method and to the cosmetic and medical use thereof, particularly due to their restorative effect on skin damage.

## Description

The present invention relates to a process for obtaining hyperozonized fatty acids from the high-degree ozonization of extra virgin sunflower oil which comprises polyunsaturated fatty acids in high concentration. Likewise, the present invention relates to the hyperozonized fatty acids obtained by said procedure and to their cosmetic and medical use, particularly for their skin damage repairing effect. Therefore, the invention could be classified in the field of chemistry, pharmaceuticals and cosmetics.

### STATE OF THE ART

Ozonized oils do not contain ozone as such, but possess the by-products derived from the reaction of ozone with the unsaturation compounds present in vegetable oils and fats, such as aldehydes and peroxidic compounds (hydroperoxides, ozonides, diperoxides, poliperoxides, among others). Mechanism of this reaction is well defined, as well as the conditions in which the reaction must develop to favour the preferential formation of some of these peroxidic compounds (Ozonation in organic chemistry, Vols. 1 and 2, Philip S. Bailey, Editors: Alfred T. Blomquist and Harry H. Wasserman, Academic Press, New York, 1978).

These peroxidic compounds, mainly the stable ozonides (1,2,4-trioxolanes) are responsible for a series of biological functions (antimicrobial effect, cicatrization, cellular regeneration, tissue antioxidant effect, among others) widely studied (Martinez Sanchez, Gregorio, 2021. Scientific rationale for the medical applications of ozonized oils, update. Ozone Therapy Global Journal Vol. 11, n° 1, pp 201-237)*.*

In the state of the art it has been described that ozonized vegetable oils have been used in the treatment of burns, lower limb ulcers due to venous insufficiency, pressure sores, acne, fistulas and surgical wounds (see, for example, the patent applications US4451480, US45916021, US984722, US2356062, US1210949).

On the other hand, the cosmetic use related to the stimulation of tissue renewal, the oxygenation of cells and tissues can be consulted in patent GB Pat. No. 820,463).

In general ozonized oils are harmless and safe products of commercial interest. Toxicological studies demonstrating this have been carried out (see, for example, Revista CENIC, Vol. 26, Special No., p 105, 1995), histological and mutagenic studies (see, for example, Revista CENIC, Biological Sciences 20 (1-2-3), 1-4 and 23, 1989), genotoxic studies (see, for example, Revista CENIC Biological Sciences 29 (3): 200, 1998) and teratogenic studies (see, for example, Proceedings of the 1st Ibero-American Congress of Ozone Applications, City of Havana, Ozone in Medicine 11, 1990; and WO 03/085072 A1).

Within these margins, ozonized oils have been described for their medical and cosmetic use (see, for example, WO 01/37829 A1 and WO 03/085072 A1) in rapid skin regeneration and cicatrization.

Given the medical and cosmetic interest of said compounds, production methods have been described which, to date, allow obtaining them by, for example, the ozonization of a water-oil emulsion 1-50% in volume, by bubbling a ozone-oxygen mixture (ozone-air) whose ozone concentration is between 1-15% in volume of gas using a scheme of 100 Litres/hour of the ozone-oxygen mixture per 500 Litres of emulsion, obtaining high peroxide indices without the need to ozonize to complete saturation the unsaturated compounds, favouring the formation of β-hydroxy-hydroperoxides, retarding the formation of polymers with stability and achieving a final product stability greater than a year for sunflower oil and of 24 months for theobroma oil (see WO 03/085072 A1).

Therefore, it would be desirable to have this new matrix of hyperozonized fatty acids which, by possessing a high concentration of stable ozonides, allow improving the effects of ozonized oils as well as greater stability, together with a simple and harmless obtaining procedure, which allows obtaining said stable compounds without increasing viscosity nor altering safety parameters, such as a high acidity index, presence of formaldehyde.

### DESCRIPTION OF THE INVENTION

In a first aspect, the invention relates to a procedure for obtaining hyperozonized fatty acids comprising the steps of:
i. concentration of the polyunsaturated fatty acids fraction of sunflower oil to obtain a sunflower oil matrix with a concentration of at least a 90% of polyunsaturated fatty acids while keeping unchanged the minority fraction that contains polyphenols, phytosterols and tocopherols, by means of fractionation by dry crystallization; and
ii. ozonization of the sunflower oil matrix of step (i) in a bubbling reactor with a gas flow of between 30% and 35% ozone from pure oxygen, at a temperature of between 25°C and 30°C, and using a gas flow rate ratio between 150 L/h and 500 L/h to obtain the hyperozonized fatty acids.

The preparation procedure of the hyperozonized fatty acids of the present invention prioritises the formation of stable ozonides (1,2,4-trioxolanes) to which greater biological activity is attributed than to β-hydroxy-hydroperoxides, achieving moreover high peroxide indices without ozonizing to complete saturation the unsaturated compounds and without the need to be an emulsion, avoiding a previous step (that of the production of an emulsion) as in the case of patent application WO 03/085072 A1, since work is carried out directly with a matrix of extra virgin sunflower oil; furthermore, it surpasses the stability of the final product both in ozonized sunflower oil and theobroma oil, since the hyperozonized fatty acids achieve a stability of 36 months; finally the hyperozonized fatty acids obtained by the ozonization procedure, retain the extra virgin nature of the sunflower oil and to maintain their stability they must be stored at no more than 30 °C.

Thus, by means of the procedure of the invention, the hyperozonized fatty acids are obtained after concentrating linoleic acid by means of a simple and harmless process known as fractionation by dry crystallization, which allows filtering by melting points the presence of saturated fatty acids and a percentage of oleic acid present in the matrix until reaching a concentration above 90% linoleic acid without affecting the presence of phytosterols, polyphenols, vitamins nor the organoleptic properties of the extra virgin sunflower oil which has a lower melting point.

In particular, the procedure of the invention is carried out by adding the extra virgin sunflower oil to a stainless steel container that is kept at -1 °C for 3 h achieving to bring to the melting point all the content of saturated fatty acids. Subsequently, the content is poured into another stainless steel container after sieving, preferably of 1.41 mm, which allows retaining the oil in solid state and extracting the oil in liquid state corresponding to the unsaturated fatty acids and to the minority fraction (composed of polyphenols, tocopherols and phytosterols). This second matrix is the one that will be ozonized.

The percentages indicated in the steps of the procedure refer both to weight and to volume of the total sunflower oil matrix.

In another embodiment the invention relates to the procedure defined above, wherein the polyunsaturated fatty acids are linoleic acid.

In another embodiment the invention relates to the procedure defined above, wherein the sunflower oil is extra virgin sunflower oil.

In the present invention the term "hyperozonized fatty acids" refers to an extra virgin matrix of sunflower oil or of other vegetable origin that after a high-degree ozonization process presents peroxide indices greater than 200 mEq prioritising stable ozonides (1,2,4-trioxolanes) immersed therein.

On the other hand, the present invention refers to that the extra virgin matrix, after the high-degree ozonization process, still presents a high concentration of linoleic acid, as well as the intact and unchanged presence of the minority fraction composed of polyphenols, sterols and tocopherols. These latter (linoleic acid and minority fraction) present a synergistic effect with the hyperozonized fatty acids that improve their medical and cosmetic properties.

The procedure of the invention comprises a step of fractionation by dry crystallization that allows filtering by melting points the presence of saturated fatty acids and a percentage of oleic acid present in the matrix until reaching a concentration greater than 90% linoleic acid without affecting the presence of phytosterols, polyphenols, vitamins nor the organoleptic properties of the extra virgin sunflower oil.

The procedure of the invention, by ozonizing a matrix rich in unsaturations in this case greater than a 90% of linoleic acid with a gas flow at 30-35% ozone, allows reaching high peroxide indices of 200 to 1200 mEq O2/Kg, and majority-wise of secondary ozonides or Criegee ozonides retarding the formation of polymers.

On the other hand, the 30-35% ozone flow applied by bubbling traverses the sunflower oil matrix highly concentrated in linoleic acid (greater than 90%) during the whole ozonization process which lasts 3 hours, using a gas flow (L/h) - Oil volume (L) ratio between 150 and 500. This high ratio allows reducing the concentration of the more volatile components during the ozonization process, by being dragged and expelled from the reactor by the gas stream and prioritising the formation of stable ozonides.

With the procedure of the invention the highly concentrated linoleic acid in the matrix is preferably ozonized to a high degree; up to reach a concentration between 200 and 1,200 mEq depending on the formulation of the final product; the reaction does not occur at total saturation of the sunflower oil matrix, so that the minority fraction of sterols, polyphenols and tocopherols of the sunflower oil matrix remains unchanged during ozonization, due to the affinity of ozone with linoleic acid.

In the previous paragraph total saturation is understood to mean total oxidation, or the total breaking of the unsaturations present in the matrix by ozone. If we ozonize to total saturation the matrix of the invention, which contains, preferably, 90% linoleic acid, after the process we would have of that 90% a 0% of unsaturations (linoleic acid).

Thus, thanks to the procedure of the invention the production of stable ozonides in high concentration is favoured without altering the extra virgin nature of the sunflower oil matrix, also without producing poliperoxides.

In the ozonization step of the invention a concentration of ozone between 30 and 35% obtained from pure oxygen (medicinal) is used in order to avoid the formation of toxic by-products derived from the nitrogen present in air, avoiding subsequent filtration processes to eliminate these toxic by-products, processes that degrade the secondary ozonides formed or that alter the extra virgin nature of the sunflower oil matrix. Therefore, the procedure of the invention has the additional advantage that it can be carried out without the need to include a filtration step since it does not generate cytotoxic by-products.

The reason why with the process of the invention said cytotoxic by-products are not generated is because the oxygen used is of medicinal origin and not from air, furthermore, because, in the saturation conditions in which it operates, other components are not oxidized, only part of the majoritarian fraction is oxidized, that is to say, linoleic acid. Thus, operating with a matrix with a high concentration of unsaturations avoids the oxidation of all the components of the matrix and the formation of toxic products.

Another aspect of the invention relates to hyperozonized fatty acids obtained by the procedure described above, characterized because they comprise peroxidic compounds, preferably in a concentration between 200 meq and 1200 meq, more preferably where the peroxidic compounds are 1,2,4 trioxolanes (Criegee ozonides), and even more preferably where the 1,2,4 trioxolanes (Criegee ozonides) are in a concentration between 200 meq and 1200 meq.

Another aspect of the present invention relates to a pharmaceutical composition comprising the hyperozonized fatty acids described above and one or more pharmaceutically acceptable excipients.

Another aspect of the invention relates to the hyperozonized fatty acids described above for use as a medicament.

Another aspect of the invention relates to the use of the hyperozonized fatty acids described above for the manufacture of a medicament.

Another aspect of the present invention relates to a method of treating a disease, in a subject in need thereof, especially in humans, which comprises administering to said subject an effective amount of the hyperozonized fatty acids described above.

Another aspect of the invention relates to the hyperozonized fatty acids described above for use in the treatment and/or prevention of a disease, for example: prevention and treatment of pressure injuries in their different stages, prevention and treatment of radiodermatitis, among other conditions that require the repair of skin damage.

Another aspect of the invention relates to the use of the hyperozonized fatty acids described above as a cosmetic.

In another embodiment the invention relates to the use of the hyperozonized fatty acids described above, wherein the cosmetic use refers to skin hydration, recovery of its elasticity and repair of photodamage.

Throughout the description and the claims the word "comprises" and its variants are not intended to exclude other technical features, additives, components or steps. For those skilled in the art, other objects, advantages and characteristics of the invention will be partly apparent from the description and partly from the practice of the invention. The following examples are provided by way of illustration, and are not intended to be limiting of the present invention.

### EXAMPLES

### Example 1. Obtaining an extra virgin matrix with a concentration greater than 90% of linoleic acid and the subsequent ozonization process to produce hyperozonized fatty acids.

Step 1. Obtaining an extra virgin matrix with a high linoleic acid content.

100 litres of extra virgin sunflower oil are placed inside a continuous stirring reactor equipped with cooling jackets that allow lowering the temperature of the lipid matrix to -1 degrees in 3 hours; after 3 hours, the content is passed through a sieve number 14 (1.41 mm) to a storage container, extracting in the process the solid material (mostly saturated fatty acids that have reached their melting point) and in the storage container of the matrix, an extra virgin sunflower oil with a content greater than 90% linoleic acid, according to gas chromatography.

Step 2. Ozonization process to produce hyperozonized fatty acids.

500 litres of extra virgin sunflower oil with high linoleic acid content (greater than 90%) are placed inside a bubbling reactor. A gas flow (ozone-oxygen) with a concentration of up to 35% ozone v/v is passed through the oil at a rate of 150 litres per hour, reactor temperature is kept at no more than 30°C. Obtaining from this ozonization scheme, at the first hour a peroxide index between 200-400 mEq mainly of stable ozonides (1,2,4-trioxolanes), at the second hour a peroxide index between 400-800 mEq of stable ozonides and the third hour a peroxide index between 800-1200 mEq of stable ozonides. The product is stored at a temperature that does not exceed 30°C.

In relation to the quality of the hyperozonized fatty acids proposed in the present invention, the following can be used as indicators: the acid index (AI), the concentration of aldehydes, the determination of viscosity and/or the peroxide index (PI) using potassium iodide according to USP XXIV and B.P. 2000. But by virtue that the present ozonization process proposed has as end point to reach parameters between 200 to 1200 mEq O2/kg, and that it is a simple procedure, the peroxide index (PI) and the acid index (AI) are used as indicators of progress and safety of the process, evaluated every hour until completing the 3 hour cycle.

Stability tests verify that the hyperozonized fatty acids obtained by means of the present invention maintain their clinical and/or cosmetic properties as the case may be, without evidence of production of toxic derivatives for 36 months.

### Example 2. Application of hyperozonized fatty acids (AGHOZ) in pressure injuries grade I and II.

The CIEC Centro Internacional de Estudios Clinicos carried out an exploratory study whose objective was to test the efficacy and safety of hyperozonized fatty acids (AGHOZ) in the healing of pressure injuries grade I and II in institutionalized elderly adult patients in Santiago de Chile. For this purpose, a clinical protocol was authorised by the Ethics Committee, nursing staff were trained, and informed consent or assent was signed as appropriate. This centre carries out all the standard pressure injury prevention measures (position change, orthoses, etc.) but despite this lesions were generated. 6 patients were included in total 3 men and 3 women, aged 72 ± 2.5 years, all bedridden, with comorbidities: 83% HTN, 67% DM2, 33% renal failure, 33% organic brain damage among others. The lesions were on bony prominences, sacrum 80% heels 30% elbows 10% (2 patients had more than 1 pressure injury) 70% of lesions corresponded to grade I pressure injuries and 30% grade **II** pressure injuries, none with signs of active infection. All patients were frail, with multiple pathologies that delay healing, and had failed usual prevention and treatment therapies for pressure injuries. The hyperozonized fatty acids (AGHOZ) were used twice a day with gentle spreading in dressings as standard measures against pressure injuries.

The daily use of hyperozonized fatty acids led to a total improvement of grade **I** pressure injuries in 10 ± 3 days and grade **II** pressure injuries in 15 ± 3 days, without related adverse events and with very good tolerance and satisfaction from the nurses in charge and the patients. The first signs of clear improvement were evidenced between the 3rd and the 7th day in all patients, which is considered excellent given the population and the conditions of the patients included in the study.

## Claims

1. Method for obtaining hyperozonized fatty acids comprising the steps of:
i. concentration of the polyunsaturated fatty acids fraction of sunflower oil to obtain a sunflower oil matrix with a concentration of at least a 90% of polyunsaturated fatty acids while keeping unchanged the minority fraction that contains polyphenols, phytosterols and tocopherols, by means of fractionation by dry crystallization; and
ii. ozonization of the sunflower oil matrix of step (i) in a bubbling reactor with a gas flow of between 30% and 35% ozone from pure oxygen, at a temperature of between 25° and 30°C, and using a gas flow rate ratio between 150 L/h and 500 L/h to obtain the hyperozonized fatty acids.

2. The method according to claim 1, wherein the polyunsaturated fatty acids are linoleic acid.

3. The method according to any of claims 1 or 2, wherein the sunflower oil is extra virgin sunflower oil.

4. Hyperozonized fatty acids obtained by the method described in any of claims 1 to 3 and characterized because they comprise peroxidic compounds in a concentration of between 200 meq and 1200 meq.

5. A pharmaceutical composition comprising the hyperozonized fatty acids according to claim 4 and one or more pharmaceutically acceptable excipients.

6. Hyperozonized fatty acids according to claim 4, for use as a medicament.

7. Hyperozonized fatty acids according to claim 4, for use in the treatment and/or prevention of a disease selected from pressure injuries in their different grades and radiodermatitis in their different grades.

8. Use of the hyperozonized fatty acids according to claim 4, as a cosmetic.

9. The use according to claim 8, wherein the cosmetic use refers to skin hydration, recovery of its elasticity and repair of photodamage.
